# EUROPEAN PATENT APPLICATION

(11) **EP 4 643 915 A1**
(43) Date of publication of application: **05.11.2025**
(21) Application number: 22969560.6
(22) Date of filing: 28.12.2022
(51) Int. Cl.: A61M 16/00

(54) **VENTILATION DEVICE, VENTILATION CONTROL METHOD THEREFOR, AND STORAGE MEDIUM**

(71) Applicant: Shenzhen Mindray Animal Medical Technology Co., Ltd., Shenzhen, Guangdong 518110 (CN)
(72) Inventor: LI, Ran, Shenzhen, Guangdong 518110 (CN); YU, Lee-Hsing, Shenzhen, Guangdong 518110 (CN)
(74) Representative: KIPA AB
(86) International application number: PCT/CN2022/142665
(87) International publication number: WO 2024/138405

(57) **Abstract**

A ventilation device, a ventilation control method therefor, and a storage medium. The method comprises: acquiring ventilation control parameters, and causing, on the basis of the ventilation control parameters, a ventilation airway assembly to ventilate a patient; acquiring a target exhalation CO₂ concentration range; acquiring an actual exhalation CO₂ concentration of the patient; determining whether the actual exhalation CO₂ concentration is within the target exhalation CO₂ concentration range: if the actual exhalation CO₂ concentration is not within the target exhalation CO₂ concentration range, adjusting the ventilation control parameters, and causing, on the basis of the adjusted ventilation control parameters, the ventilation airway assembly to ventilate the patient, so that according to the comparison result of the actual exhalation CO₂ concentration of the patient and the target exhalation CO₂ concentration range, the pulmonary ventilation state of the patient can be automatically, quickly, and accurately determined and the ventilation control parameters can be adjusted.

## Description

### TECHNICAL FIELD

The disclosure relates to the field of pulmonary ventilation, and in particular to a ventilation device and a ventilation control method therefor, and a storage medium.

### BACKGROUND

Human breathing refers to the periodic and rhythmic inhalation and exhalation of gas to absorb oxygen and expel carbon dioxide, thereby achieving gas exchange. When some patients are unable to breathe spontaneously, mechanical ventilation can help the patients breathe. For example, if a patient cannot breathe spontaneously, an external device such as a ventilator is generally used to provide respiratory support for the patient. It can be seen that mechanical ventilation refers to a ventilation method in which a mechanical apparatus is used to replace, control, or modify the patient's spontaneous breathing movement. Such a mechanical apparatus may be generally referred to as a ventilation device.

During the ventilation process of the ventilation device, ventilation control parameters for controlling the ventilation of the ventilation device need to be preset. If the ventilation control parameters are not set appropriately, the pulmonary ventilation of the patient may be in the state of hyperventilation or hypoventilation, which, if not detected and corrected in a timely manner over a prolonged period, will cause serious physical damage to the patient. In the prior art, a user monitors ventilation parameters indicating the pulmonary ventilation state in real time and, when hyperventilation or hypoventilation is detected, manually adjusts ventilation control parameters to correct hyperventilation or hypoventilation, which is not conducive to the rapid and accurate detection and correction of hypoventilation or hyperventilation.

### SUMMARY

In order to solve the above problems, the disclosure provides a ventilation device and a ventilation control method therefor, and a storage medium.

According to a first aspect, a ventilation device is provided according to an embodiment. The ventilation device includes:
a ventilation path assembly configured to ventilate a patient; and
a controller configured to:
   acquire ventilation control parameters and cause, on the basis of the ventilation control parameters, the ventilation path assembly to ventilate the patient, where the ventilation control parameters include a tidal volume and/or trigger sensitivity, the trigger sensitivity being a sensitivity of triggering the ventilation path assembly to deliver gas to the patient;
   acquire a target exhalation CO₂ concentration range, the target exhalation CO₂ concentration range being a range corresponding to an exhalation CO₂ concentration of the patient when the patient is in a normal pulmonary ventilation state;
   acquire an actual exhalation CO₂ concentration of the patient;
   determine whether the actual exhalation CO₂ concentration is within the target exhalation CO₂ concentration range; and
   if the actual exhalation CO₂ concentration is not within the target exhalation CO₂ concentration range, adjust the ventilation control parameters and cause, on the basis of the adjusted ventilation control parameters, the ventilation path assembly to ventilate the patient.

In one embodiment, the ventilation device further includes:
a respiration monitoring module configured to monitor a state change in an oral cavity of the patient during spontaneous breathing;
the controller is configured to identify an inspiration trigger moment of the patient according to a relationship between the monitored state change in the oral cavity of the patient during spontaneous breathing and the trigger sensitivity, and to control the ventilation path assembly to deliver gas to the patient at the inspiration trigger moment, such that the ventilation device achieves human-machine synchronization during the delivery of the gas to the patient.

In one embodiment, the state change includes a pressure change and/or a flow rate change.

In one embodiment, the pressure change includes one or more of speed, trend and magnitude of change in negative pressure in the oral cavity of the patient;
the flow rate change includes one or more of speed, trend and magnitude of change in flow rate of air in the oral cavity of the patient.

In one embodiment, the trigger sensitivity includes a trigger pressure threshold and/or a trigger flow rate threshold;
determining an inspiration trigger moment of the patient according to the relationship between the monitored state change in the oral cavity of the patient during spontaneous breathing and the trigger sensitivity includes:
identifying that the patient is at the inspiration trigger moment when the pressure change reaches the trigger pressure threshold;
and/or identifying that the patient is at the inspiration trigger moment when the flow rate change reaches the trigger flow rate threshold.

In one embodiment, if the actual exhalation CO₂ concentration is not within the target exhalation CO₂ concentration range, adjusting the ventilation control parameters includes:
if the actual exhalation CO₂ concentration is not within the target exhalation CO₂ concentration range, adjusting the ventilation control parameters and acquiring a deviation of the actual exhalation CO₂ concentration from the target exhalation CO₂ concentration range; and
determining a time interval and an adjustment step size for a next adjustment of the ventilation control parameters on the basis of the deviation of the actual exhalation CO₂ concentration from the target exhalation CO₂ concentration range.

In one embodiment, the time interval for the next adjustment of the ventilation control parameters is negatively correlated with the deviation of the actual exhalation CO₂ concentration from the target exhalation CO₂ concentration range; and
the adjustment step size for the next adjustment of the ventilation control parameters is positively correlated with the deviation of the actual exhalation CO₂ concentration from the target exhalation CO₂ concentration range.

In one embodiment, the ventilation control parameters further include a respiratory rate to characterize a frequency at which the ventilation path assembly delivers the gas to the patient.

In one embodiment, if the actual exhalation CO₂ concentration is not within the target exhalation CO₂ concentration range, adjusting the ventilation control parameters further includes:
if the actual exhalation CO₂ concentration is lower than the target exhalation CO₂ concentration range, reducing the tidal volume, and/or adjusting the trigger sensitivity to trigger the ventilation path assembly less sensitively to deliver the gas to the patient, and/or reducing the respiratory rate; and
if the actual exhalation CO₂ concentration is higher than the target exhalation CO₂ concentration range, increasing the tidal volume, and/or adjusting the trigger sensitivity to trigger the ventilation path assembly more sensitively to deliver the gas to the patient, and/or increasing the respiratory rate.

In one embodiment, each of the ventilation control parameters has a preset parameter range; and adjusting the ventilation control parameters further includes:
adjusting the ventilation control parameters within the preset parameter range.

According to a second aspect, a ventilation control method for a ventilation device is provided according to an embodiment, the method including:
acquiring ventilation control parameters and causing, on the basis of the ventilation control parameters, the ventilation path assembly to ventilate the patient, where the ventilation control parameters include a tidal volume and/or trigger sensitivity, the trigger sensitivity being a sensitivity of triggering the ventilation path assembly to deliver gas to the patient;
acquire a target exhalation CO₂ concentration range, the target exhalation CO₂ concentration range being a range corresponding to an exhalation CO₂ concentration of the patient when the patient is in a normal pulmonary ventilation state;
acquire an actual exhalation CO₂ concentration of the patient;
determine whether the actual exhalation CO₂ concentration is within the target exhalation CO₂ concentration range; and
if the actual exhalation CO₂ concentration is not within the target exhalation CO₂ concentration range, adjust the ventilation control parameters and cause, on the basis of the adjusted ventilation control parameters, the ventilation path assembly to ventilate the patient.

In one embodiment, the ventilation device further includes:
acquiring a state change in an oral cavity of the patient during spontaneous breathing; and
identifying an inspiration trigger moment of the patient according to a relationship between the state change in the oral cavity of the patient during spontaneous breathing and the trigger sensitivity and causing the ventilation path assembly to deliver the gas to the patient at the inspiration trigger moment, such that the ventilation device achieves human-machine synchronization during the delivery of the gas to the patient.

In one embodiment, the state change includes a pressure change and/or a flow rate change.

In one embodiment, the pressure change includes one or more of speed, trend and magnitude of change in negative pressure in the oral cavity of the patient;
the flow rate change includes one or more of speed, trend and magnitude of change in flow rate of air in the oral cavity of the patient.

In one embodiment, the trigger sensitivity includes a trigger pressure threshold and/or a trigger flow rate threshold;
determining an inspiration trigger moment of the patient according to the relationship between the state change in the oral cavity of the patient during spontaneous breathing and the trigger sensitivity includes:
identifying that the patient is at the inspiration trigger moment when the pressure change reaches the trigger pressure threshold;
and/or identifying that the patient is at the inspiration trigger moment when the flow rate change reaches the trigger flow rate threshold.

In one embodiment, if the actual exhalation CO₂ concentration is not within the target exhalation CO₂ concentration range, adjusting the ventilation control parameters includes:
if the actual exhalation CO₂ concentration is not within the target exhalation CO₂ concentration range, adjusting the ventilation control parameters and acquiring a deviation of the actual exhalation CO₂ concentration from the target exhalation CO₂ concentration range; and
determining a time interval and an adjustment step size for a next adjustment of the ventilation control parameters on the basis of the deviation of the actual exhalation CO₂ concentration from the target exhalation CO₂ concentration range.

In one embodiment, the time interval for the next adjustment of the ventilation control parameters is negatively correlated with the deviation of the actual exhalation CO₂ concentration from the target exhalation CO₂ concentration range; and
the adjustment step size for the next adjustment of the ventilation control parameters is positively correlated with the deviation of the actual exhalation CO₂ concentration from the target exhalation CO₂ concentration range.

In one embodiment, the ventilation control parameters further include a respiratory rate to characterize a frequency at which the ventilation path assembly delivers the gas to the patient.

In one embodiment, if the actual exhalation CO₂ concentration is not within the target exhalation CO₂ concentration range, adjusting the ventilation control parameters further includes:
if the actual exhalation CO₂ concentration is lower than the target exhalation CO₂ concentration range, reducing the tidal volume, and/or adjusting the trigger sensitivity to trigger the ventilation path assembly less sensitively to deliver the gas to the patient, and/or reducing the respiratory rate; and
if the actual exhalation CO₂ concentration is higher than the target exhalation CO₂ concentration range, increasing the tidal volume, and/or adjusting the trigger sensitivity to trigger the ventilation path assembly more sensitively to deliver the gas to the patient, and/or increasing the respiratory rate.

In one embodiment, each of the ventilation control parameters has a preset parameter range; and adjusting the ventilation control parameters further includes:
adjusting the ventilation control parameters within the preset parameter range.

According to the above embodiments, a ventilation device and a ventilation control method therefor, and a storage medium are provided. The method includes: acquiring ventilation control parameters, causing, on the basis of the ventilation control parameters, a ventilation path assembly to ventilate a patient, acquiring a target exhalation CO₂ concentration range, acquiring an actual exhalation CO₂ concentration of the patient, determining whether the actual exhalation CO₂ concentration is within the target exhalation CO₂ concentration range, and if the actual exhalation CO₂ concentration is not within the target exhalation CO₂ concentration range, adjusting the ventilation control parameters and causing, on the basis of the adjusted ventilation control parameters, the ventilation path assembly to ventilate the patient. In this way, according to the comparison result of the actual exhalation CO₂ concentration of the patient and the target exhalation CO₂ concentration range, the pulmonary ventilation state of the patient can be automatically, quickly, and accurately determined and the ventilation control parameters can be adjusted.

### BRIEF DESCRIPTION OF THE DRAWINGS

FIG. 1 is a structural schematic diagram of a ventilation device according to an embodiment of the disclosure;
FIG. 2 is a specific structural schematic diagram of a ventilation device according to an embodiment of the disclosure;
FIG. 3 is a specific structural schematic diagram of a ventilation device according to another embodiment of the disclosure;
FIG. 4 is a structural schematic diagram of a ventilation device according to another embodiment of the disclosure;
FIG. 5 is a flowchart of a control method for a ventilation device according to an embodiment of the disclosure; and
FIG. 6 is a flowchart of a control method for a ventilation device according to another embodiment of the disclosure.

### DESCRIPTION OF THE PREFERRED EMBODIMENTS

The disclosure will be further described in detail below through specific implementations in conjunction with the accompanying drawings. Associated similar element reference numerals are used for similar elements in different implementations. In the following implementations, many details are described such that the disclosure may be better understood. However, it may be effortlessly appreciated by persons skilled in the art that some of the features may be omitted, or may be substituted by other elements, materials, and methods in different cases. In certain cases, some operations involved in the disclosure are not displayed or described in the specification, which is to prevent a core part of the disclosure from being obscured by too much description. Moreover, for persons skilled in the art, a detailed description of the involved operations is not necessary, and the involved operations can be thoroughly understood according to the description in the specification and general technical knowledge of the art.

In addition, the characteristics, operations, or features described in the specification may be combined in any appropriate manner to form various implementations. In addition, the steps or actions in the method description may also be exchanged or adjusted in order in a way that is obvious to persons skilled in the art. Therefore, the various orders in the specification and the accompanying drawings are merely for the purpose of a clear description of a certain embodiment and are not meant to be a necessary order unless it is otherwise stated that a certain order must be followed.

The serial numbers themselves for the components herein, for example, "first", "second", etc. are merely used to distinguish the described objects, and do not have any sequential or technical meaning. Moreover, as used in the disclosure, "connection" or "coupling", unless otherwise stated, includes both direct and indirect connections (couplings).

The parameters referred to in the disclosure are described below.

Tidal volume refers to the volume of gas that is delivered to a patient by the ventilation device during each respiratory cycle.

Trigger sensitivity refers to a parameter threshold that triggers the ventilation device to deliver gas to the patient.

Respiratory rate refers to a frequency at which the ventilation device delivers gas to the patient.

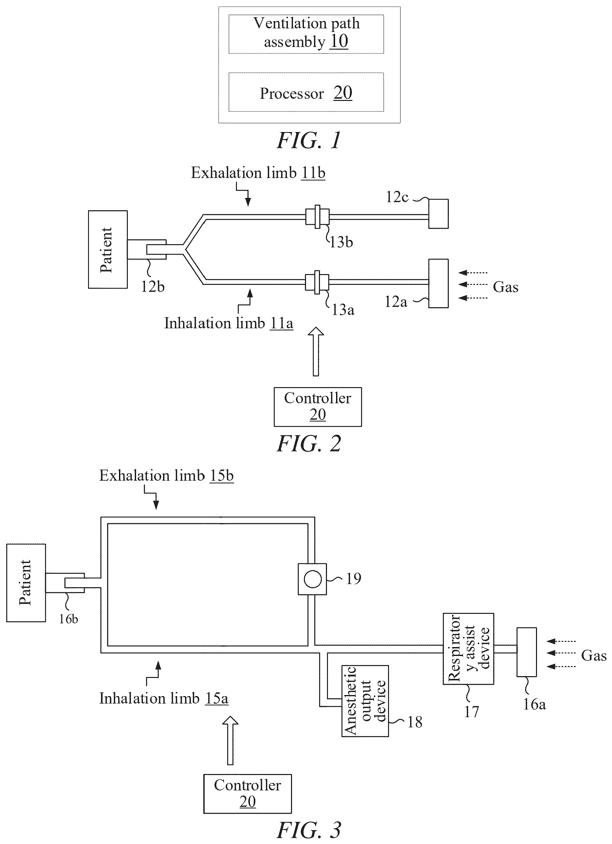

Referring to FIG. 1, a ventilation device is provided according to some embodiments of the disclosure. The ventilation device may include a ventilation path assembly 10 and a controller 20. In some embodiments, the ventilation path assembly 10 is configured to input gas to the patient and/or to discharge the patient's exhaled gas. It should be noted that the gas supplied by the ventilation path assembly 10 to the patient may originate from within the ventilation device, for example, gas for breathing that is provided by an internal gas supply of the ventilation device to the ventilation path assembly 10, such as gas delivered to the patient by a built-in turbine; and the gas supplied by the ventilation path assembly 10 to the patient may also be supplied from an external gas supply.

In some embodiments, the ventilation device may be a ventilator. In some embodiments, the ventilation device may also be an anesthesia machine. The detailed description is given below.

The ventilation device according to some embodiments may be a ventilator which is an artificial mechanical ventilation device to assist or control spontaneous breathing movements of a patient to achieve the function of gas exchange in the lungs, and reduce the physiological workload of the body so as to facilitate the recovery of respiratory function. Referring to FIG. 2, in some embodiments, the ventilation path assembly 10 may include an inhalation limb 11a, an exhalation limb 11b, a gas supply interface 12a, a breathing interface 12b, an exhaust interface 12c, an inhalation controller 13a, and an exhalation controller 13b, as detailed below.

In some embodiments, the gas supply interface 12a is configured to be connected to a gas supply (not depicted) which supplies gas that is typically oxygen, a mixture of oxygen and air, etc. The gas supply may be an internal gas supply of the ventilation device and may also be an external gas supply, for example, the external gas supply may be a compressed gas cylinder or a central gas supply.

In some embodiments, the breathing interface 12b is configured to connect the patient to the inhalation limb 11a and the exhalation limb 11b; the breathing interface 12b may direct the gas delivered by the inhalation limb 11a to the patient, and may also direct the gas exhaled by the patient through the exhalation limb 11b to the exhaust interface 12c. In some embodiments, the breathing interface 12b may be a nasal cannula or a mask worn over the mouth and nose, which may be determined according to actual demands.

In some embodiments, the exhaust interface 12c may be in communication with an external environment or in communication with a dedicated gas recovery apparatus (not depicted).

In some embodiments, the inhalation limb 11a is connected between the breathing interface 12b and the gas supply interface 12a and configured to supply gas, such as oxygen or air, to the patient. For example, gas input from the gas supply interface 12a enters the inhalation limb 11a and then enters the lungs of the patient via the breathing interface 12b. In some embodiments, the inhalation controller 13a is arranged on the inhalation limb 11a and configured to control the gas delivered from the gas supply interface 12a to the patient through the inhalation limb 11a, for example, open the inhalation limb 11a or close the inhalation limb 11a according to instructions from the processor 20, or to control the flow rate or pressure of the gas in the inhalation limb 11a. In some embodiments, the inhalation controller 13a may include one or more devices capable of controlling flow or pressure, such as an inspiratory valve, a one-way valve, or a flow controller.

In some embodiments, the exhalation limb 11b is connected between the breathing interface 12b and the exhaust interface 12c and configured to direct the gas exhaled by the patient to the exhaust interface 12c. In some embodiments, the exhalation controller 13b is arranged on the exhalation limb 11b and configured to open the exhalation limb 11b or close the exhalation limb 11b according to instructions from the processor 20, or to control the flow rate or pressure of the exhaled gas from the patient. In some embodiments, the exhalation controller 13b may include one or more devices capable of controlling flow or pressure, such as an expiratory valve, a one-way valve, or a flow controller.

The foregoing provides description of the ventilation device implemented as a ventilator. In some embodiments, the ventilation device may also be an anesthesia machine which is primarily configured to supply anesthetic gas, deliver the anesthetic gas to a respiratory system of the patient via a respirator, and control the amount of anesthetic gas inhaled by the patient. Referring to FIG. 3, in some embodiments, the ventilation path assembly 10 may include an inhalation limb 15a, an exhalation limb 15b, a gas supply interface 16a, a breathing interface 16b, a respiratory assist component 17, and an anesthetic output component 18; and in some embodiments, the ventilation path assembly 10 may further include a gas recovery component 19, as described in detail below.

The gas supply interface 16a is configured to be connected to a gas supply (not depicted) which is configured to supply gas. In one embodiment, the gas supplied by the gas supply may include oxygen, nitrous oxide (laughing gas), and/or air, etc.

The respiratory assist component 17 is configured to provide driving force for non-spontaneous breathing of the patient and maintain the airway unobstructed. In some embodiments, the respiratory assist component 17 controls delivery of the gas supplied by the gas supply to the patient through the inhalation limb 15a. In some specific embodiments, fresh gas introduced via the gas supply interface 16a, exhaled gas from the patient through the exhalation limb 15b, and an anesthetic that is output by the anesthetic output component 18 are mixed by the respiratory assist component 17, and are then delivered to the breathing interface 16b through the inhalation limb 15a to enable the patient to inhale, and the exhaled gas from the patient is received through the exhalation limb 15b.

The anesthetic output component 18 is configured to supply an anesthetic. Typically, the anesthetic is mixed in a gaseous form with the fresh air introduced via the gas supply interface 16a, and the mixture is delivered into a breathing circuit. In some specific embodiments, the anesthetic output component 18 may be implemented as an anesthetic vaporizer. The anesthetic is usually in a liquid form and is stored in the anesthetic vaporizer. Optionally, a heating apparatus may be provided in the anesthetic vaporizer to heat and volatilize the anesthetic to generate anesthetic vapor. The anesthetic output component 18 is in communication with a pipeline of the gas supply interface 16a, so that the anesthetic vapor and the fresh air introduced via the gas supply interface 16a are mixed and then delivered together into the inhalation limb 15a.

In some embodiments, the inhalation limb 15a is in communication with the exhalation limb 15b to form a closed circuit, and the gas recovery component 19 is arranged on a pipeline of the exhalation limb 15b. The mixture of fresh air introduced via the gas supply interface 16a is input via an inlet of the inhalation limb 15a and delivered to the patient via the breathing interface 16b arranged at an outlet of the inhalation limb 15a. In some embodiments, the breathing interface 16b may be a mask, a nasal cannula, or a tracheal cannula. An inlet of the exhalation limb 15b is in communication with the breathing interface 16b. When the patient exhales, the exhaled gas enters the gas recovery component 19 through the exhalation limb 15b, carbon dioxide in the exhaled gas is removed by a substance in the gas recovery component 19. The gas after carbon dioxide is removed is recirculated into the inhalation limb 15a.

The foregoing provides a description of the ventilation device implemented as an anesthesia machine.

In some embodiments of the disclosure, according to the comparison result of the actual exhalation CO₂ concentration of the patient and the target exhalation CO₂ concentration range, the ventilation device can determine the pulmonary ventilation state of the patient automatically, quickly, and accurately and adjust the ventilation control parameters.

In some embodiments, the controller 20 is configured to acquire ventilation control parameters and cause, on the basis of the ventilation control parameters, a ventilation path assembly to ventilate the patient; acquire a target exhalation CO₂ concentration range; acquire an actual exhalation CO₂ concentration of the patient; determine whether the actual exhalation CO₂ concentration is within the target exhalation CO₂ concentration range; and if the actual exhalation CO₂ concentration is not within the target exhalation CO₂ concentration range, adjust the ventilation control parameters and cause, on the basis of the adjusted ventilation control parameters, the ventilation path assembly 10 to ventilate the patient. In some embodiments, the ventilation control parameters include a tidal volume and/or trigger sensitivity.

In some embodiments, the target exhalation CO₂ concentration range refers to a range corresponding to an exhalation CO₂ concentration of the patient when the pulmonary ventilation state of the patient is normal. The target exhalation CO₂ concentration range includes a target exhalation CO₂ concentration upper limit and a target exhalation CO₂ concentration lower limit. The target exhalation CO₂ concentration upper limit and the target exhalation CO₂ concentration lower limit may be preset by a user or may be data stored in a storage medium of the controller 20.

In some embodiments, the actual exhalation CO₂ concentration of the patient may be obtained by monitoring the exhalation CO₂ concentration of the patient by a gas analyzer. In some embodiments, the gas analyzer may be a CO₂ analyzer. The CO₂ analyzer is connected to the exhalation limb (11b or 15b) of the ventilation path assembly, and extracts part of the gas exhaled by the patient through the exhalation limb (11b or 15b) for analysis of the CO₂ concentration. In some embodiments, both the target exhalation CO₂ concentration and the actual exhalation CO₂ concentration refer to an end-expiratory CO₂ concentration.

In some embodiments, when the actual exhalation CO₂ concentration is within the target exhalation CO₂ concentration range, no adjustment of the current ventilation control parameters is required, and the ventilation path assembly 10 is controlled to continue to ventilate the patient according to the current ventilation control parameters.

In some embodiments, when the actual exhalation CO₂ concentration is not within the target exhalation CO₂ concentration range, the ventilation control parameters need to be cyclically adjusted multiple times until the adjusted ventilation control parameters cause the actual exhalation CO₂ concentration to be within the target exhalation CO₂ concentration range.

In some embodiments, if the actual exhalation CO₂ concentration is not within the target exhalation CO₂ concentration range, the ventilation control parameters are adjusted, and a deviation of the actual exhalation CO₂ concentration from the target exhalation CO₂ concentration range is acquired; and on the basis of the deviation of the actual exhalation CO₂ concentration from the target exhalation CO₂ concentration range, a time interval and an adjustment step size for a next adjustment of the ventilation control parameters are determined. The time interval for the next adjustment of the ventilation control parameters is a time interval for a next cycle, and the adjustment step size is the parameter size for each adjustment of the ventilation control parameters.

In some embodiments, the time interval for the next adjustment of the ventilation control parameters is negatively correlated with the deviation of the actual exhalation CO₂ concentration from the target exhalation CO₂ concentration range; and the adjustment step size for the next adjustment of the ventilation control parameters is positively correlated with the deviation of the actual exhalation CO₂ concentration from the target exhalation CO₂ concentration range. In other words, the greater the deviation of the actual exhalation CO₂ concentration from the target exhalation CO₂ concentration range, the smaller the time interval for the next adjustment of the ventilation control parameters, and the greater the adjustment step size for the next adjustment of the ventilation control parameters. For example, when the deviation of the actual exhalation CO₂ concentration from the target exhalation CO₂ concentration range is not large, the time interval for the next adjustment of the ventilation control parameters may be 2 min, and the ventilation control parameters are adjusted according to one adjustment step size; and when the deviation of the actual exhalation CO₂ concentration from the target exhalation CO₂ concentration range is large, the time interval for the next adjustment of the ventilation control parameters may be 30 s, and the ventilation control parameters are adjusted according to three adjustment step sizes. In other embodiments, in this embodiment, the ventilation control parameters may also be adjusted according to an existing feedback control adjustment algorithm which may be a PID control algorithm, a PI control algorithm, etc. and may be adjusted according to actual conditions, which will not be repeated herein.

The following provides a detailed description of the process of cyclically adjusting the ventilation control parameters multiple times when the actual exhalation CO₂ concentration is within the target exhalation CO₂ concentration range.

At an initial parameter step, the controller 20 acquires initial ventilation control parameters and causes, on the basis of the initial ventilation control parameters, the ventilation path assembly to ventilate a patient.

At a first adjustment step, after an initial time interval, an actual exhalation CO₂ concentration of the patient is acquired, whether the actual exhalation CO₂ concentration is within the target exhalation CO₂ concentration range is determined, if the actual exhalation CO₂ concentration is not within the target exhalation CO₂ concentration range, a tidal volume and/or trigger sensitivity in the initial ventilation control parameters are adjusted according to an initial adjustment step size to obtain the ventilation control parameters after the first adjustment, and on the basis of the ventilation control parameters after the first adjustment, the ventilation path assembly 10 is controlled to ventilate the patient; and meanwhile, a deviation of the actual exhalation CO₂ concentration from the target exhalation CO₂ concentration range is acquired, and on the basis of the deviation of the actual exhalation CO₂ concentration from the target exhalation CO₂ concentration range, a time interval and an adjustment step size for a second adjustment of the ventilation control parameters are determined and denoted as a second time interval and a second adjustment step size.

At a second adjustment step, after a second time interval, the actual exhalation CO₂ concentration of the patient is acquired, whether the actual exhalation CO₂ concentration is not within the target exhalation CO₂ concentration range is determined, if the actual exhalation CO₂ concentration is not within the target exhalation CO₂ concentration range, the tidal volume and/or the trigger sensitivity in the first adjusted ventilation control parameters are adjusted according to a second adjustment step size to obtain the ventilation control parameters after the second adjustment, and on the basis of the ventilation control parameters after the second adjustment, the ventilation path assembly 10 is controlled to ventilate the patient; and meanwhile, a deviation of the actual exhalation CO₂ concentration from the target exhalation CO₂ concentration range is acquired, and on the basis of the deviation of the actual exhalation CO₂ concentration from the target exhalation CO₂ concentration range, a time interval and an adjustment step size for a third adjustment of the ventilation control parameters are determined and denoted as a third time interval and a third adjustment step size.

By analogy, at an nth adjustment step, after an nth time interval, the actual exhalation CO₂ concentration of the patient is acquired, whether the actual exhalation CO₂ concentration is not within the target exhalation CO₂ concentration range is determined, if the actual exhalation CO₂ concentration is within the target exhalation CO₂ concentration range, no adjustment of the ventilation control parameters after the (n-1)th adjustment is performed, and on the basis of the ventilation control parameters after the (n-1)th adjustment, the ventilation path assembly 10 is controlled to ventilate the patient.

After n-1 adjustments of the ventilation control parameters, the actual exhalation CO₂ concentration acquired on the basis of the ventilation control parameters after the (n-1)th adjustment is within the target exhalation CO₂ concentration range, and at this point, no further adjustment of the ventilation control parameters is performed, that is, the adjustment process of the ventilation control parameters is completed.

In some embodiments, the ventilation control parameters may further include a respiratory rate to characterize the number of times per minute that the ventilation device delivers the gas to the patient. Since adjusting the tidal volume and/or the trigger sensitivity may have a certain impact on the respiratory rate, in this embodiment it is possible to adjust the respiratory rate while adjusting the tidal volume and/or the trigger sensitivity.

In some embodiments, if the actual exhalation CO₂ concentration is not within the target exhalation CO₂ concentration range, the tidal volume and/or the trigger sensitivity and/or the respiratory rate in the ventilation control parameters are adjusted as follows.

If the actual exhalation CO₂ concentration is below the target exhalation CO₂ concentration range, the tidal volume is reduced and/or the trigger sensitivity is adjusted, so as to trigger the ventilation path assembly less sensitively to deliver the gas to the patient and/or to reduce the respiratory rate. If the actual exhalation CO₂ concentration is higher than the target exhalation CO₂ concentration range, the tidal volume is increased and/or the trigger sensitivity is adjusted, so as to trigger the ventilation path assembly more sensitively to deliver the gas to the patient and/or increase the respiratory rate.

The principle of adjusting the ventilation control parameters as described above is illustrated in this embodiment by taking a ventilator as an example.

The greater the amount of gas delivered by the ventilator per breath, the higher the oxygen concentration in the lungs of the patient, and the more CO₂ is exhaled. As a result, the amount of CO₂ retained in the body of the patient decreases, causing the amount of CO₂ exhaled to progressively decrease. If the actual exhalation CO₂ concentration is detected to be lower than the target exhalation CO₂ concentration range, that is, the actual exhalation CO₂ concentration is lower than the target exhalation CO₂ concentration lower limit, which indicates that the amount of CO₂ retained in the body of the patient is too low, and further indicates that the volume of gas delivered by the ventilator in each breath is too large. Therefore, it is necessary to reduce the volume of gas delivered per breath of the ventilator, that is, to decrease the tidal volume, reduce the respiratory rate, and adjust the trigger sensitivity to be less sensitive. Conversely, if the actual exhalation CO₂ concentration is detected to be higher than the target exhalation CO₂ concentration range, that is, the actual exhalation CO₂ concentration is higher than the target exhalation CO₂ concentration upper limit, which indicates that the amount of CO₂ retained in the body of the patient is too high. Therefore, it is necessary to increase the volume of gas delivered per breath of the ventilator, that is, to increase the tidal volume, increase the respiratory rate, and adjust the trigger sensitivity to be more sensitive.

It should be noted that each ventilation control parameter has a preset parameter range. During the adjustment of the ventilation control parameters, the adjustments should be made within the respective preset parameter ranges. If any parameter exceeds the preset parameter range, the adjustment of the ventilation control parameter should be stopped. The tidal volume, the trigger sensitivity and the respiratory rate each have a corresponding preset parameter range.

In summary, according to the embodiments of the disclosure, according to the comparison result of the actual exhalation CO₂ concentration and the target exhalation CO₂ concentration range, it is possible to determine a pulmonary respiratory state of the patient to automatically adjust the breathing control parameters.

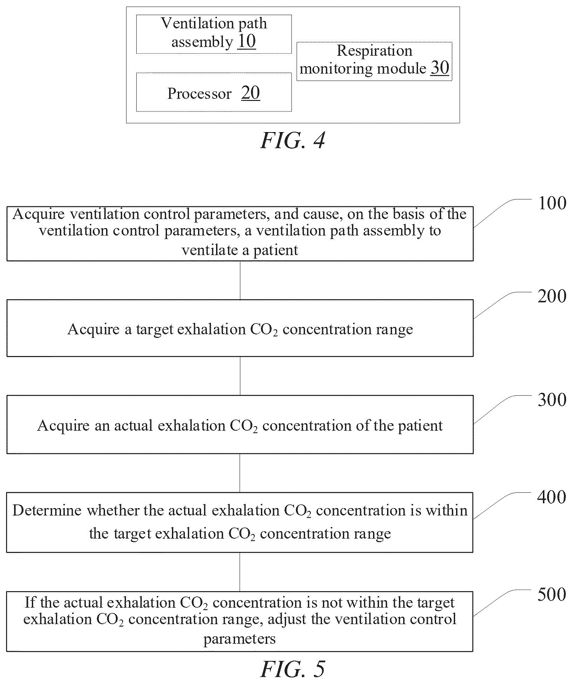

Referring to FIG. 4, a ventilation device according to the embodiments of the disclosure further includes: a respiration monitoring module 30.

The respiration monitoring module 30 is configured to monitor a state change in an oral cavity of a patient during spontaneous breathing. The state change includes a pressure change and/or a flow rate change.

The controller 20 is configured to identify the inspiration trigger moment of the patient according to a relationship between the monitored state change in the oral cavity of the patient during spontaneous breathing and the trigger sensitivity, and to control the ventilation path assembly to deliver gas to the patient at the inspiration trigger moment, such that the ventilation device achieves human-machine synchronization during the delivery of the gas to the patient.

In some embodiments, the pressure change includes one or more of speed, trend and magnitude of change in negative pressure in the oral cavity of the patient. In this case, the inspiration trigger moment of the patient may be identified according to the relationship between one or more changes in negative pressure in the oral cavity and the trigger sensitivity. After the inspiration trigger moment is identified, the ventilation path assembly is controlled to deliver the gas to the patient, which can help the patient inhale according to the inhalation intention of the patient.

In some embodiments, the speed of change in negative pressure in the oral cavity refers to the rate at which a change value of the negative pressure in the oral cavity increases or decreases. The trend of change in negative pressure in the oral cavity is a change trend of the negative pressure in the oral cavity. The magnitude of change in negative pressure in the oral cavity refers to a change value of the negative pressure in the oral cavity, which may sometimes be a difference in negative pressure of the oral cavity and may also be a difference between a measured oral negative pressure and a preset oral negative pressure.

In some embodiments, the trigger sensitivity includes a trigger pressure threshold. Determining an inspiration trigger moment of the patient according to the relationship between the monitored state change in the oral cavity of the patient during spontaneous breathing and the trigger sensitivity includes: identifying that the patient is at the inspiration trigger moment when the pressure change reaches the trigger pressure threshold. The pressure change reaching the trigger pressure threshold includes: identifying that the patient is at the inspiration trigger moment when the pressure change exhibits an upward trend and the magnitude of the pressure change reaches the trigger pressure threshold; or identifying that the patient is at the inspiration trigger moment when the speed of the pressure change reaches the trigger pressure threshold; or identifying that the patient is at the inspiration trigger moment when the magnitude of the pressure change reaches the trigger pressure threshold.

In some embodiments, the flow rate change includes one or more of speed, trend and magnitude of change in flow rate of air in the oral cavity of the patient. In this case, the inspiration trigger moment of the patient may be identified according to the relationship between one or more changes in flow rate of air in the oral cavity and the trigger sensitivity. After the inspiration trigger moment is identified, the ventilation path assembly is controlled to deliver the gas to the patient, which can help the patient inhale according to the inhalation intention of the patient.

In some embodiments, the speed of change in flow rate of air in the oral cavity refers to the rate at which a change value of the flow rate of air in the oral cavity increases or decreases. The trend of change in flow rate of air in the oral cavity refers to a change trend of the flow rate of air in the oral cavity. The magnitude of change in flow rate of air in the oral cavity refers to the change value of the flow rate of air in the oral cavity, which may sometimes be a difference in flow rate of air in the oral cavity and may also be a difference between a measured flow rate of air in the oral cavity and a preset flow rate of air in the oral cavity.

In some embodiments, the trigger sensitivity includes a trigger flow rate threshold. Determining an inspiration trigger moment of the patient according to the relationship between the monitored state change in the oral cavity of the patient during spontaneous breathing and the trigger sensitivity includes: identifying that the patient is at the inspiration trigger moment when the change in flow rate of air in the oral cavity reaches the trigger pressure threshold. The change in flow rate of air in the oral cavity reaching the trigger pressure threshold includes: identifying that the patient is at the inspiration trigger moment when the change in flow rate of air in the oral cavity exhibits an upward trend and the magnitude of pressure change reaches the trigger pressure threshold; or identifying that the patient is at the inspiration trigger moment when the speed of change in flow rate of air in the oral cavity reaches the trigger pressure threshold; or identifying that the patient is at the inspiration trigger moment when the magnitude of change in flow rate of air in the oral cavity reaches the trigger pressure threshold.

In some embodiments, the trigger sensitivity is a variable threshold. In this embodiment, the trigger sensitivity may be adjusted according to a comparison result between the actual exhalation CO₂ concentration and the target exhalation CO₂ concentration range. When the actual exhalation CO₂ concentration of the patient is lower than the target exhalation CO₂ concentration range, the trigger sensitivity is increased. When the actual exhalation CO₂ concentration of the patient is higher than the target exhalation CO₂ concentration range, the trigger sensitivity is reduced. Thus, under the control of the adjusted trigger sensitivity, the ventilation device can maintain the actual exhalation CO₂ concentration of the patient within the target exhalation CO₂ concentration range and also deliver the gas when the patient has an inhalation intention, such that the ventilation device achieves human-machine synchronization during delivery of the gas to the patient.

In some embodiments, the change in negative pressure in the oral cavity may be measured by a pressure sensor and the change in flow rate of air in the oral cavity may be measured by a flow sensor.

In some embodiments, inhaled gas provided by the ventilation device to the patient may be oxygen, and may also be a mixture of air and oxygen, and this embodiment is not limited thereto. The inhaled gas may also be a mixture of helium and oxygen. The inhaled gas may be, for example, compressed gas provided by a central gas supply system in a hospital or may be gas from the environment.

The ventilation device according to this embodiment further includes a display apparatus (not depicted). In some embodiments, the controller 20 controls the display apparatus to display a corresponding pulmonary ventilation state diagram at a display interface of the ventilation device. At least curves (a curve corresponding to the target exhalation CO₂ concentration upper limit and a curve corresponding to the target exhalation CO₂ concentration upper limit) corresponding to the target exhalation CO₂ concentration range and a fitted curve corresponding to the actual exhalation CO₂ concentration of the patient are shown in the pulmonary ventilation state diagram. In other embodiments, the controller 20 also controls the display apparatus to display a pressure curve corresponding to a negative pressure in the oral cavity of the patient and/or a flow rate curve corresponding to a flow rate of air in the oral cavity of the patient on the interface of the ventilation device.

Some embodiments of the disclosure also disclose a ventilation control method, and the ventilation control method according to some embodiments is applied to the ventilation device according to some embodiments of the disclosure.

Referring to FIG. 5, the ventilation control method according to some embodiments includes the following steps:
step 100: acquiring ventilation control parameters and causing, on the basis of the ventilation control parameters, a ventilation path assembly to ventilate a patient, where the ventilation control parameters include a tidal volume and/or trigger sensitivity, the trigger sensitivity being a sensitivity that triggers the ventilation path assembly to deliver gas to the patient;
step 200: acquiring a target exhalation CO₂ concentration range, the target exhalation CO₂ concentration range being a range corresponding to an exhalation CO₂ concentration of the patient when the patient is in a normal pulmonary ventilation state;
step 300: acquiring an actual exhalation CO₂ concentration of the patient;
step 400: determining whether the actual exhalation CO₂ concentration is within the target exhalation CO₂ concentration range; and
step 500: if the actual exhalation CO₂ concentration is not within the target exhalation CO₂ concentration range, adjusting the ventilation control parameters and causing, on the basis of the adjusted ventilation control parameters, the ventilation path assembly to deliver gas to the patient.

In some embodiments, at step 500, when the actual exhalation CO₂ concentration is within the target exhalation CO₂ concentration range, adjusting the ventilation control parameters includes: when the actual exhalation CO₂ concentration is not within the target exhalation CO₂ concentration range, cyclically adjusting the ventilation control parameters multiple times until the adjusted ventilation control parameters cause the actual exhalation CO₂ concentration to be within the target exhalation CO₂ concentration range.

The ventilation control parameters are cyclically adjusted multiple times as follows.

At an initial parameter step, the controller 20 acquires initial ventilation control parameters and causes, on the basis of the initial ventilation control parameters, the ventilation path assembly to ventilate a patient.

At a first adjustment step, after an initial time interval, an actual exhalation CO₂ concentration of the patient is acquired, whether the actual exhalation CO₂ concentration is within the target exhalation CO₂ concentration range is determined, if the actual exhalation CO₂ concentration is not within the target exhalation CO₂ concentration range, a tidal volume and/or trigger sensitivity in the initial ventilation control parameters are adjusted according to an initial adjustment step size to obtain the ventilation control parameters after the first adjustment, and on the basis of the ventilation control parameters after the first adjustment, the ventilation path assembly 10 is controlled to ventilate the patient; and meanwhile, a deviation of the actual exhalation CO₂ concentration from the target exhalation CO₂ concentration range is acquired, and on the basis of the deviation of the actual exhalation CO₂ concentration from the target exhalation CO₂ concentration range, a time interval and an adjustment step size for a second adjustment of the ventilation control parameters are determined and denoted as a second time interval and a second adjustment step size.

At a second adjustment step, after a second time interval, the actual exhalation CO₂ concentration of the patient is acquired, whether the actual exhalation CO₂ concentration is not within the target exhalation CO₂ concentration range is determined, if the actual exhalation CO₂ concentration is not within the target exhalation CO₂ concentration range, the tidal volume and/or the trigger sensitivity in the first adjusted ventilation control parameters are adjusted according to a second adjustment step size to obtain the ventilation control parameters after the second adjustment, and on the basis of the ventilation control parameters after the second adjustment, the ventilation path assembly 10 is controlled to ventilate the patient; and meanwhile, a deviation of the actual exhalation CO₂ concentration from the target exhalation CO₂ concentration range is acquired, and on the basis of the deviation of the actual exhalation CO₂ concentration from the target exhalation CO₂ concentration range, a time interval and an adjustment step size for a third adjustment of the ventilation control parameters are determined and denoted as a third time interval and a third adjustment step size.

By analogy, at an nth adjustment step, after an nth time interval, the actual exhalation CO₂ concentration of the patient is acquired, whether the actual exhalation CO₂ concentration is not within the target exhalation CO₂ concentration range is determined, if the actual exhalation CO₂ concentration is within the target exhalation CO₂ concentration range, no adjustment of the ventilation control parameters after the (n-1)th adjustment is performed, and on the basis of the ventilation control parameters after the (n-1)th adjustment, the ventilation path assembly 10 is controlled to ventilate the patient.

After n-1 adjustments of the ventilation control parameters, the actual exhalation CO₂ concentration acquired on the basis of the ventilation control parameters after the (n-1)th adjustment is within the target exhalation CO₂ concentration range, and at this point, no further adjustment of the ventilation control parameters is performed, that is, the adjustment process of the ventilation control parameters is completed.

In some embodiments, the ventilation control parameters may further include a respiratory rate to characterize the number of times per minute that the ventilation device delivers the gas to the patient. Since adjusting the tidal volume and/or the trigger sensitivity may have a certain impact on the respiratory rate, in this embodiment it is possible to adjust the respiratory rate while adjusting the tidal volume and/or the trigger sensitivity.

In some embodiments, at step 500, if the actual exhalation CO₂ concentration is not within the target exhalation CO₂ concentration range, the ventilation control parameters are adjusted according to the following adjustment principles.

If the actual exhalation CO₂ concentration is below the target exhalation CO₂ concentration range, the tidal volume is reduced and/or the trigger sensitivity is adjusted, so as to trigger the ventilation path assembly less sensitively to deliver the gas to the patient and/or to reduce the respiratory rate. If the actual exhalation CO₂ concentration is higher than the target exhalation CO₂ concentration range, the tidal volume is increased and/or the trigger sensitivity is adjusted, so as to trigger the ventilation path assembly more sensitively to deliver the gas to the patient and/or increase the respiratory rate.

The foregoing provides a description of adjusting the tidal volume and/or the trigger sensitivity according to the comparison result between the actual exhalation CO₂ concentration and the target exhalation CO₂ concentration range.

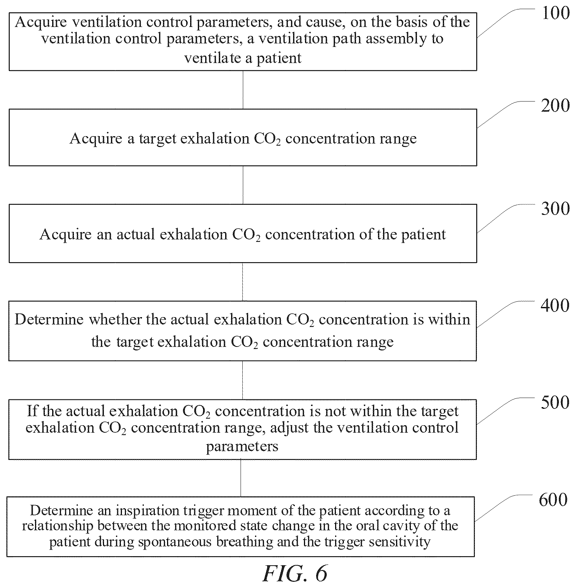

Referring to FIG. 6, the ventilation control method according to some embodiments further includes the following step:
step 600: according to the relationship between the monitored state change in the oral cavity of the patient during spontaneous breathing and the trigger sensitivity, identifying the inspiration trigger moment of the patient, and causing the ventilation path assembly 10 to deliver the gas to the patient at the inspiration trigger moment, such that the ventilation device achieves human-machine synchronization during delivery of the gas to the patient.

In some embodiments, the state change in the oral cavity of the patient during spontaneous breathing is monitored and acquired by the respiration monitoring module 30 in the ventilation device.

In some embodiments, the pressure change includes one or more of speed, trend and magnitude of change in negative pressure in the oral cavity of the patient. The trigger sensitivity includes a trigger pressure threshold and a trigger flow rate threshold.

In some embodiments, when the speed of pressure change measured by the respiration monitoring module 30 increases from a value near zero, it is identified that the patient is at the inspiration trigger moment. That is, when a pressure gradient value exceeds the zero and gradually increases, it is identified that the patient is at the inspiration trigger moment. In this case, the inspiration trigger moment of the patient can be accurately identified.

In some embodiments, the inspiration trigger moment is identified according to the magnitude of the pressure change and the trend of the pressure change obtained by the respiration monitoring module 30. Specifically, when the pressure change measured by the respiration monitoring module 30 exhibits an upward trend and the magnitude of the pressure change reaches the trigger pressure threshold, it is identified that the patient is at the inspiration trigger moment. In this case, the inspiration trigger moment of the patient can be accurately identified on the basis of the magnitude and trend of the pressure change.

In some embodiments, the flow rate change includes one or more of speed, trend and magnitude of change in flow rate of air in the oral cavity of the patient. The trigger sensitivity includes a trigger flow rate threshold.

In some embodiments, when the speed of change in flow rate of air in the oral cavity measured by the respiration monitoring module 30 increases from a value near zero, it is identified that the patient is at the inspiration trigger moment. That is, when a flow rate gradient value exceeds zero and gradually increases, it is identified that the patient is at the inspiration trigger moment. In this case, the inspiration trigger moment of the patient can be accurately identified.

In some embodiments, the inspiration trigger moment is identified according to the magnitude of change in flow rate of air and the trend of change in flow rate of air obtained by the respiration monitoring module 30. Specifically, when the change in flow rate of air measured by the respiration monitoring module 30 exhibits an upward trend and the magnitude of change in flow rate of air reaches the trigger flow rate threshold, it is identified that the patient is at the inspiration trigger moment. In this case, the inspiration trigger moment of the patient can be accurately identified on the basis of the magnitude and trend of change in flow rate of air.

The forgoing provides a description of identifying the inspiration trigger moment of the patient according to the adjusted trigger sensitivity and the state change in the oral cavity of the patient during spontaneous breathing, such that the ventilation device achieves human-machine synchronization during the delivery of the gas to the patient.

Descriptions are provided herein with reference to various exemplary embodiments. However, those skilled in the art should understand that variations and corrections may be made to the exemplary embodiments without departing from the scope of this specification. For example, various operational steps and assemblies for executing the operational steps may be implemented in different methods according to specific applications or in consideration of any number of cost functions associated with operations of the system (for example, one or more steps may be deleted, modified or incorporated into other steps).

Those skilled in the art may understand that all or some of functions of various methods in the foregoing implementations may be implemented through hardware or a computer program. When all or some of the functions in the above implementations are implemented by means of a computer program, the program may be stored in a computer-readable storage medium, and the storage medium may include: a read-only memory, a random access memory, a magnetic disk, an optical disk, a hard disk, and the like, and the program is executed by a computer to implement the above functions. For example, the program is stored in a memory of a device, and when the program in the memory is executed by a processor, all or some of the foregoing functions may be implemented. In addition, when all or some of the functions in the foregoing implementations are implemented through a computer program, the program may alternatively be stored in a storage medium such as a server, another computer, a magnetic disk, an optical disc, a flash drive, or a removable hard disk, and downloaded or replicated for storage in a memory of a local device or version update is performed on a system of the local device. When a processor executes the program in the memory, all or some of the functions in the foregoing implementations may be implemented.

The disclosure has been described above with respect to specific examples, which are merely for the purpose of facilitating understanding of the disclosure and are not intended to limit the disclosure. For persons skilled in the technical field to which the disclosure pertains, several simple deductions, variations, or replacements may also be made according to the idea of the disclosure.

## Claims

1. A ventilation device, **characterized by** comprising:
a ventilation path assembly configured to ventilate a patient; and
a controller configured to:
acquire ventilation control parameters and cause, on the basis of the ventilation control parameters, the ventilation path assembly to ventilate the patient, wherein the ventilation control parameters comprise a tidal volume and/or trigger sensitivity, the trigger sensitivity being a sensitivity of triggering the ventilation path assembly to deliver gas to the patient;
acquire a target exhalation CO₂ concentration range, the target exhalation CO₂ concentration range being a range corresponding to an exhalation CO₂ concentration of the patient when the patient is in a normal pulmonary ventilation state;
acquire an actual exhalation CO₂ concentration of the patient;
determine whether the actual exhalation CO₂ concentration is within the target exhalation CO₂ concentration range; and
if the actual exhalation CO₂ concentration is not within the target exhalation CO₂ concentration range, adjust the ventilation control parameters and cause, on the basis of the adjusted ventilation control parameters, the ventilation path assembly to ventilate the patient.

2. The ventilation device of claim 1, further comprising:
a respiration monitoring module configured to monitor a state change in an oral cavity of the patient during spontaneous breathing;
wherein the controller is configured to identify an inspiration trigger moment of the patient according to a relationship between the monitored state change in the oral cavity of the patient during spontaneous breathing and the trigger sensitivity, and to control the ventilation path assembly to deliver gas to the patient at the inspiration trigger moment, such that the ventilation device achieves human-machine synchronization during the delivery of the gas to the patient.

3. The ventilation device of claim 2, **characterized in that** the state change comprises a pressure change and/or a flow rate change.

4. The ventilation device of claim 3, **characterized in that** the pressure change comprises one or more of speed, trend and magnitude of change in negative pressure in the oral cavity of the patient; and
the flow rate change comprises one or more of speed, trend and magnitude of change in flow rate of air in the oral cavity of the patient.

5. The ventilation device of claim 3 or 4, **characterized in that** the trigger sensitivity comprises a trigger pressure threshold and/or a trigger flow rate threshold; and
determining an inspiration trigger moment of the patient according to the relationship between the monitored state change in the oral cavity of the patient during spontaneous breathing and the trigger sensitivity comprises:
identifying that the patient is at the inspiration trigger moment when the pressure change reaches the trigger pressure threshold;
and/or identifying that the patient is at the inspiration trigger moment when the flow rate change reaches the trigger flow rate threshold.

6. The ventilation device of any one of claims 1 to 5, **characterized in that** if the actual exhalation CO₂ concentration is not within the target exhalation CO₂ concentration range, adjusting the ventilation control parameters comprises:
if the actual exhalation CO₂ concentration is not within the target exhalation CO₂ concentration range, adjusting the ventilation control parameters and acquiring a deviation of the actual exhalation CO₂ concentration from the target exhalation CO₂ concentration range; and
determining a time interval and an adjustment step size for a next adjustment of the ventilation control parameters on the basis of the deviation of the actual exhalation CO₂ concentration from the target exhalation CO₂ concentration range.

7. The ventilation device of claim 6, **characterized in that** the time interval for the next adjustment of the ventilation control parameters is negatively correlated with the deviation of the actual exhalation CO₂ concentration from the target exhalation CO₂ concentration range; and
the adjustment step size for the next adjustment of the ventilation control parameters is positively correlated with the deviation of the actual exhalation CO₂ concentration from the target exhalation CO₂ concentration range.

8. The ventilation device of any one of claims 1 to 7, **characterized in that** the ventilation control parameters further comprise a respiratory rate to characterize a frequency at which the ventilation path assembly delivers the gas to the patient.

9. The ventilation device of claim 8, **characterized in that** if the actual exhalation CO₂ concentration is not within the target exhalation CO₂ concentration range, adjusting the ventilation control parameters further comprises:
if the actual exhalation CO₂ concentration is lower than the target exhalation CO₂ concentration range, reducing the tidal volume, and/or adjusting the trigger sensitivity to trigger the ventilation path assembly less sensitively to deliver the gas to the patient, and/or reducing the respiratory rate; and
if the actual exhalation CO₂ concentration is higher than the target exhalation CO₂ concentration range, increasing the tidal volume, and/or adjusting the trigger sensitivity to trigger the ventilation path assembly more sensitively to deliver the gas to the patient, and/or increasing the respiratory rate.

10. The ventilation device of any one of claims 1 to 9, **characterized in that** each of the ventilation control parameters has a preset parameter range; and adjusting the ventilation control parameters further comprises:
adjusting the ventilation control parameters within the preset parameter range.

11. A ventilation control method for a ventilation device, comprising:
acquiring ventilation control parameters and causing, on the basis of the ventilation control parameters, the ventilation path assembly to ventilate the patient, wherein the ventilation control parameters comprise a tidal volume and/or trigger sensitivity, the trigger sensitivity being a sensitivity of triggering the ventilation path assembly to deliver gas to the patient;
acquiring a target exhalation CO₂ concentration range, the target exhalation CO₂ concentration range being a range corresponding to an exhalation CO₂ concentration of the patient when the patient is in a normal pulmonary ventilation state;
acquiring an actual exhalation CO₂ concentration of the patient;
determining whether the actual exhalation CO₂ concentration is within the target exhalation CO₂ concentration range; and
if the actual exhalation CO₂ concentration is not within the target exhalation CO₂ concentration range, adjusting the ventilation control parameters and causing, on the basis of the adjusted ventilation control parameters, the ventilation path assembly to ventilate the patient.

12. The method of claim 11, further comprising:
acquiring a state change in an oral cavity of the patient during spontaneous breathing; and
identifying an inspiration trigger moment of the patient according to a relationship between the state change in the oral cavity of the patient during spontaneous breathing and the trigger sensitivity and causing the ventilation path assembly to deliver the gas to the patient at the inspiration trigger moment, such that the ventilation device achieves human-machine synchronization during the delivery of the gas to the patient.

13. The method of claim 12, **characterized in that** the state change comprises a pressure change and/or a flow rate change.

14. The method of claim 13, **characterized in that** the pressure change comprises one or more of speed, trend and magnitude of change in negative pressure in the oral cavity of the patient; and
the flow rate change comprises one or more of speed, trend and magnitude of change in flow rate of air in the oral cavity of the patient.

15. The method of claim 13 or 14, **characterized in that** the trigger sensitivity comprises a trigger pressure threshold and/or a trigger flow rate threshold; and
determining an inspiration trigger moment of the patient according to the relationship between the state change in the oral cavity of the patient during spontaneous breathing and the trigger sensitivity comprises:
identifying that the patient is at the inspiration trigger moment when the pressure change reaches the trigger pressure threshold;
and/or identifying that the patient is at the inspiration trigger moment when the flow rate change reaches the trigger flow rate threshold.

16. The method of any one of claims 11 to 15, **characterized in that** if the actual exhalation CO₂ concentration is not within the target exhalation CO₂ concentration range, adjusting the ventilation control parameters comprises:
if the actual exhalation CO₂ concentration is not within the target exhalation CO₂ concentration range, adjusting the ventilation control parameters and acquiring a deviation of the actual exhalation CO₂ concentration from the target exhalation CO₂ concentration range; and
determining a time interval and an adjustment step size for a next adjustment of the ventilation control parameters on the basis of the deviation of the actual exhalation CO₂ concentration from the target exhalation CO₂ concentration range.

17. The method of claim 16, **characterized in that** the time interval for the next adjustment of the ventilation control parameters is negatively correlated with the deviation of the actual exhalation CO₂ concentration from the target exhalation CO₂ concentration range; and
the adjustment step size for the next adjustment of the ventilation control parameters is positively correlated with the deviation of the actual exhalation CO₂ concentration from the target exhalation CO₂ concentration range.

18. The method of any one of claims 11 to 17, **characterized in that** the ventilation control parameters further comprise a respiratory rate to characterize a frequency at which the ventilation path assembly delivers the gas to the patient.

19. The method of claim 18, **characterized in that** if the actual exhalation CO₂ concentration is not within the target exhalation CO₂ concentration range, adjusting the ventilation control parameters further comprises:
if the actual exhalation CO₂ concentration is lower than the target exhalation CO₂ concentration range, reducing the tidal volume, and/or adjusting the trigger sensitivity to trigger the ventilation path assembly less sensitively to deliver the gas to the patient, and/or reducing the respiratory rate; and
if the actual exhalation CO₂ concentration is higher than the target exhalation CO₂ concentration range, increasing the tidal volume, and/or adjusting the trigger sensitivity to trigger the ventilation path assembly more sensitively to deliver the gas to the patient, and/or increasing the respiratory rate.

20. The method of any one of claims 11 to 19, **characterized in that** each of the ventilation control parameters has a preset parameter range; and adjusting the ventilation control parameters further comprises:
adjusting the ventilation control parameters within the preset parameter range.
